# EUROPEAN PATENT APPLICATION

(11) **EP 4 684 784 A1**
(43) Date of publication of application: **28.01.2026**
(21) Application number: 24865932.8
(22) Date of filing: 20.08.2024
(51) Int. Cl.: A61K 31/19, A61P 29/00, A61P 39/06, A61K 8/365, A61Q 19/00

(54) **ANTI-INFLAMMATORY COSMETIC COMPOSITION**

(30) Priority: 12.09.2023 KR 20230120962; 26.07.2024 KR 20240099305; 26.07.2024 KR 20240099306
(71) Applicant: LG Chem, Ltd., Seoul 07336 (KR)
(72) Inventor: PARK, Hun, Daejeon 34122 (KR); JUNG, Woochul, Daejeon 34122 (KR); YANG, Jungil, Daejeon 34122 (KR); KANG, Donggyun, Daejeon 34122 (KR)
(74) Representative: Plasseraud IP
(86) International application number: PCT/KR2024/096044
(87) International publication number: WO 2025/058493

(57) **Abstract**

The present disclosure relates to a cosmetic composition having anti-inflammatory activity. According to the present disclosure, a cosmetic composition that can inhibit the production of reactive nitrogen species, which is an inflammatory mediator involved in the induction of inflammation in cells, is provided.

## Description

### [TECHNICAL FIELD]

### CROSS-REFERENCE TO RELATED APPLICATION(S)

This application claims priority to and the benefit of Korean Patent Application No. 10-2023-0120962, filed on September 12, 2023, Korean Patent Application No. 10-2024-0099305, filed on July 26, 2024, and Korean Patent Application No. 10-2024-0099306, filed on July 26, 2024, with the Korean Intellectual Property Office, the disclosures of which are incorporated herein by reference in their entirety.

The present disclosure relates to a cosmetic composition exhibiting anti-inflammatory effects.

### [BACKGROUND OF ART]

A group of oxidants containing nitrogen atom(N) are present in human body, which are called reactive nitrogen species(RNS). Reactive nitrogen species is sometimes classified into an independent species, but is generally classified into a sub-group of reactive oxygen species (ROS).

The ROS, which has a crucial role in human body and is first produced in mitochondria, is superoxide. Superoxide is converted to hydrogen peroxide which has a relatively weak oxidizing power and has less effect on cells due to enzymes, wherein hydrogen peroxide may also interact with metal ions to produce hydroxyl radicals.

The most basic reactive nitrogen species is nitric oxide(NO). Nitric oxide, synthesized by NO synthase(NOS) in the human body, is a neurotransmitter and is involved in blood pressure regulation and vascular reconnection. Nitric oxide is also associated with immune responses and the development of chronic inflammatory diseases such as rheumatoid arthritis. Nitric oxide may also react with oxygen and superoxide to generate other reactive nitrogen species such as nitrate(NO₃⁻), nitrite(NO₂⁻), peroxynitrite(ONOO⁻), and 3-nitrotyrosine. In this way, reactive nitrogen species is generated from the activity of normal cells and function to regulate cell proliferation and differentiation, etc.

However, reactive nitrogen species is produced more frequently during the inflammatory process and is an inflammatory mediator important in acute and chronic inflammation. Reactive nitrogen species functions as intracellular signal regulator, but when the concentration exceeds the normal level, it causes cell damage. It is known that when reactive nitrogen species increases excessively due to oxidative stress in cells in environments such as infrared rays, viral infections, and hyperglycemia, it may act as a cause of inflammatory diseases such as cancer and diabetes.

Therefore, consumer's request for products exhibiting anti-inflammatory effects, such as preventing, treating, and ameliorating symptoms caused by the induction of inflammation in cells, is constantly increasing.

### [DETAILED DESCRIPTION OF THE INVENTION]

### [Technical Problem]

It is an object of the present disclosure to provide a cosmetic composition having anti-inflammatory activity.

It is another object of the present disclosure to provide a pharmaceutical composition having anti-inflammatory activity.

### [Technical Solution]

According to an embodiment of the present disclosure, there is provided a cosmetic composition having anti-inflammatory activity, comprising 3-hydroxypropionic acid or a derivative thereof as an effective ingredient.

According to another embodiment of the present disclosure, there is provided use of 3-hydroxypropionic acid or a derivative thereof for the preparation of cosmetics having anti-inflammatory activity.

According to yet another embodiment of the present disclosure, there is provided a method of inhibiting the production of reactive nitrogen species, which is an inflammatory mediator, by using a cosmetic composition comprising 3-hydroxypropionic acid or a derivative thereof as an active ingredient.

According to yet another embodiment of the present disclosure, there is provided a pharmaceutical composition having anti-inflammatory activity, comprising 3-hydroxypropionic acid or a derivative thereof as an effective ingredient.

According to yet another embodiment of the present disclosure, there is provided use of 3-hydroxypropionic acid or a derivative thereof for the preparation of medicines having anti-inflammatory activity.

According to yet another embodiment of the present disclosure, there is provided a method of inhibiting the production of reactive nitrogen species, which is an inflammatory mediator, by using a pharmaceutical composition comprising 3-hydroxypropionic acid or a derivative thereof as an active ingredient.

Hereinafter, a cosmetic composition having anti-inflammatory activity or a pharmaceutical composition having anti-inflammatory activity according to specific embodiments of the present disclosure will be described in more detail.

Terms or words used in the present specification and claims should not be construed as limited to ordinary or dictionary terms, and the present disclosure should be construed with meanings and concepts that are consistent with the technical idea of the present disclosure based on the principle that the inventors may appropriately define concepts of the terms to appropriately describe their own invention in the best way.

Unless otherwise defined, all technical terms and scientific terms used in the specification have the general meaning understood by those skilled in the art to which the present disclosure belongs. The terms used in the specification are only to effectively describe a specific embodiment, but are not intended to limit the present disclosure.

While the present disclosure may be modified in various ways and take on various alternative forms, specific embodiments thereof are described in detail below. However, it should be understood that there is no intent to limit the present disclosure to the particular forms disclosed, but on the contrary, the present disclosure covers all modifications, equivalents, and alternatives falling within the spirit and scope of the present disclosure.

Singular terms used in the specification include plural terms, unless the context clearly indicates otherwise.

The terms "comprise," "include", "have", etc. are used herein to specify the presence of stated features, numbers, regions, steps, operations, elements, and/or components, but do not preclude the presence or addition of one or more other features, regions, numbers, steps, operations, elements, components, and/or groups thereof.

In describing temporal relationships, for example, when the temporal order is described using "after", "subsequent", "next", or "before", the case of a noncontinuous relationship may be included, unless "just" or "directly" is used.

As used herein, the term "derivative(s)" means a compound that has been modified from their parent organic compound by introduction of functional groups, oxidation, reduction, substitution of atoms, etc., to the extent that the structure and properties of the parent compound are not significantly changed.

As used herein, the term "comprising as an effective ingredient" means that the composition according to an embodiment of the present disclosure comprises 3-hydroxypropionic acid or a derivative thereof in an amount sufficient to achieve an anti-inflammatory effect.

As used herein, the term "anti-inflammatory" means alleviating or interrupting an inflammatory response or suppressing the process by which inflammation occurs.

As used herein, the term "prevention" means inhibiting the occurrence of symptoms caused by active oxygen. As used herein, the term "treatment" means alleviating or eliminating the appearance of symptoms caused by active oxygen that have already occurred. Further, as used herein, the term "amelioration" means alleviating or mitigating symptoms caused by active oxygen.

As a result of continuous research, the present inventors have found that 3-hydroxypropionic acid can exhibit excellent anti-inflammatory effects such as preventing, alleviating, or ameliorating symptoms caused by the induction of inflammation in cells.

In particular, 3-hydroxypropionic acid can inhibit the production of reactive nitrogen species, which is an inflammatory mediator involved in the induction of inflammation in cells, whereby a cosmetic composition comprising 3-hydroxypropionic acid or a derivative thereof as an effective ingredient can exhibit excellent anti-inflammatory effects.

According to an embodiment of the present disclosure, a cosmetic composition having anti-inflammatory activity, comprising 3-hydroxypropionic acid or a derivative thereof as an effective ingredient is provided.

The cosmetic composition having anti-inflammatory activity comprises 3-hydroxypropionic acid or a derivative thereof as an effective ingredient. Preferably, the cosmetic composition having anti-inflammatory activity comprises 3-hydroxypropionic acid as an effective ingredient.

3-Hydroxypropionic acid synthesized by a chemical method or a biological method can be applied as an active ingredient.

According to an embodiment, 3-hydroxypropionic acid obtained through a biological process such as fermenting a strain having 3-hydroxypropionic acid production ability can be preferably used as the effective ingredient. The 3-hydroxypropionic acid obtained by the above method can include a radioactive carbon isotope (¹⁴C).

Preferably, the effective ingredient may have a radioactive carbon isotope content of 20 pMC(percent modern carbon) or more and a biocarbon content of 20 wt.% or more as measured according to ASTM D6866-21 standard. Specifically, the effective ingredient may have a radioactive carbon isotope content of 20 pMC or more, or 50 pMC or more, or 90 pMC or more, or 100 pMC or more, or 90 to 110 pMC and a biocarbon content of 20 wt.% or more, or 50 wt.% or more, or 80 wt.% or more, or 90 wt.% or more, or 95 wt.% or more, or 90 to 100 wt.% as measured according to ASTM D6866-21 standard.

The radioactive carbon isotope content (pMC) refers to a ratio of the radioactive carbon isotope(¹⁴C) contained in 3-hydroxypropionic acid to the radioactive carbon isotope(¹⁴C) of the modern reference material. For reference, the effectiveness of a nuclear test program in the 1950s is continuing and not extinguished, and thus the radioactive carbon isotope content (pMC) may be greater than 100%.

The biocarbon content represents the content of biocarbon with respect to a total carbon content included in 3-hydroxypropionic acid, which may mean that a higher value can correspond to an eco-friendly compound.

If the radioactive carbon isotope content (pMC) and biocarbon content of the 3-hydroxypropionic acid are too low, eco-friendliness can be reduced and it may not be regarded as a bio-derived material.

The radioactive isotope (¹⁴C) can be nearly one per 1X10¹² carbon atoms in the earth's atmosphere and have a half-life of about 5,700 years, and it may be abundant in an upper atmosphere due to a nuclear reaction involving cosmic rays and common nitrogen (¹⁴N).

In fossil fuels, radioactive isotopes have decayed long ago and thus a ¹⁴C ratio can be substantially zero. When bio-derived materials are used as a raw material for 3-hydroxypropionic acid or when fossil fuels are used together, a content of radioactive carbon isotopes (pMC) contained in 3-hydroxypropionic acid and a content of biocarbon can be measured according to ASTM D6866-21 standard.

For example, carbon atoms included in the compound to be measured can be made into the form of graphite or carbon dioxide gas, and then measured with a mass spectrometer, or according to a liquid scintillation spectroscopy. In this case, two isotopes can be separated using an accelerator for separating ¹⁴C ions from ¹²C ions, and thus the content and content ratio can be measured with a mass spectrometer.

The cosmetic composition can inhibit the production of reactive nitrogen species, which is an inflammatory mediator due to the action of the effective ingredient. Thereby, the cosmetic composition can exhibit excellent anti-inflammatory effects, such as preventing or ameliorating symptoms caused by the induction of inflammation in cells.

Here, the active nitrogen species is at least one compound selected from the group consisting of nitric oxide(NO), nitrate(NO₃⁻), nitrite(NO₂⁻), peroxynitrite(ONOO⁻), and 3-nitrotyrtyrosine.

According to an embodiment, the cosmetic composition can contain 0.01 wt.% to 1.5 wt.% of the effective ingredient based on the total weight of the cosmetic composition.

Specifically, the cosmetic composition can contain 0.01 wt.% or more or 0.012 wt.% or more; and 1.5 wt.% or less, or 1.25 wt.% or less, or 1.1 wt.% or less, or 1.0 wt.% or less of the effective ingredient based on the total weight of the cosmetic composition.

In order to ensure that the anti-inflammatory effect can be sufficiently achieved by the action of the effective ingredient, the cosmetic composition preferably contains 0.01 wt.% or more or 0.012 wt.% or more of the effective ingredient.

However, if the effective ingredient is contained in an excessive amount, the anti-inflammatory effect may be rather reduced or side effects such as cytotoxicity may appear. Therefore, the cosmetic composition preferably contains 1.5 wt.% or less, or 1.25 wt.% or less, or 1.1 wt.% or less, or 1.0 wt.% or less of the effective ingredient.

Preferably, the cosmetic composition may contain 0.01 wt.% to 1.5 wt.%, or 0.01 wt.% to 1.25 wt.%, or 0.01 wt.% to 1.1 wt.%, or 0.01 wt.% to 1.0 wt.%, or 0.012 wt.% to 1.0 wt.% of the effective ingredient based on the total weight of the cosmetic composition.

According to an embodiment, the cosmetic composition may have a conventional cosmetic formulation in the technical field to which the present disclosure belongs. The cosmetic composition may be formulated in in a wide variety of forms, for example, including a solution, a suspension, an emulsion, a paste, a gel, a cream, a lotion, a powder, an oil, a foam, a pack, a spray, an aerosol, a balm, a solid soap, a liquid soap, a powder foundation, an emulsion foundation, or a wax foundation.

According to an embodiment, the cosmetic composition may, in addition to the effective ingredient, contain an organic solvent, a fat substance, a solubilizer, a concentrator, a gelling agent, a softener, an antioxidant, a suspending agent, a stabilizer, a forming agent, a fragrance, a surfactant, water, emulsifier, a filler, a chelating agent, preservative, vitamin, humectant, dye, pigment, hydrophilic or lipophilic activator, and the like. Further, the cosmetic composition may contain an adjuvant commonly used in the cosmetology or dermatology fields.

The cosmetic composition may further comprise a physiologically acceptable medium comprising water or a mixture of water and an organic solvent.

As an example, the organic solvent may include C1-C4 lower alcohols such as acetone, ethanol and isopropyl alcohol, alkylene glycols such as ethylene glycol and propylene glycol, ethylene glycol monomethyl ether, monoethyl ether, monobutyl ether, propylene glycol, and the like. The physiologically acceptable medium may be applied in an amount of 1 to 90 wt.% based on the total weight of the cosmetic composition.

The medium can be thickened using a thickener commonly used in cosmetics or pharmaceuticals. The thickener can be applied in an amount of 0.1 to 5 wt.% based on the total weight of the composition.

In addition, according to some embodiments of the cosmetic composition, a person skilled in the art can select adjuvants that are generally required for preparing such compositions. As an example, the adjuvants may comprise preservatives, stabilizers, pH regulators, osmotic pressure modifiers, emulsifiers, sunscreens, antioxidants, fragrances, dyes, anionic surfactants, cationic surfactants, nonionic surfactants, and amphoteric surfactants.

According to an embodiment, when the formulation of the cosmetic composition is a powder or spray, it may comprise carrier components such as lactose, talc, silica, aluminum hydroxide, calcium silicate, polyamide powder, and the like.

According to an embodiment, when the formulation of the cosmetic composition is a paste, gel, or cream, it may comprise carrier components such as animal fats, vegetable oils, waxes, paraffins, starch, tragacanth, cellulose derivatives, polyethylene glycols, silicones, bentonite, silica, talc, zinc oxide, and the like.

According to an embodiment, when the formulation of the cosmetic composition is a solution and emulsion, it may comprise carrier components such as solvent, solubilizer and emulsifier. For example, the formulation of solutions and emulsions may comprise water, ethanol, isopropanol, ethyl carbonate, ethyl acetate, benzyl alcohol, benzyl benzoate, propylene glycol, 1.3-butyleneglycol oils, glycerol fatty acid ester, polyethylene glycol or fatty acid ester of sorbitan, and the like.

According to an embodiment, when the formulation of the cosmetic composition is a suspension, it may comprise liquid diluents such as water, ethanol or propylene glycol; suspending agents such as ethoxylated isostearyl alcohols, polyoxyethylene sorbitol esters and polyoxy ethylene sorbitan esters; microcrystalline cellulose, aluminum metahydroxide, bentonite, and the like.

According to an embodiment, when the formulation of the cosmetic composition is a cleansing agent containing a surfactant, it may comprise carrier components such as an aliphatic alcohol sulfate, aliphatic alcohol ether sulfate, sulfosuccinic acid monoester, isethionate, imidazolinium derivative, sodium methyl cocoyl taurate, sodium lauroyl sarcosinate, fatty acid amide ether sulfate, alkylamidobetaine, fatty alcohol, fatty acid glyceride, fatty acid diethanolamide, vegetable oil, lanolin derivative, ethoxylated glycerol fatty acid ester, and the like.

According to another embodiment of the present disclosure, there is provided use of 3-hydroxypropionic acid or a derivative thereof for the preparation of cosmetics having anti-inflammatory activity.

Preferably, the use of 3-hydroxypropionic acid or a derivative thereof for the preparation of the above-mentioned cosmetic composition having anti-inflammatory activity is provided.

3-Hydroxypropionic acid has the effect of inhibiting the production of reactive nitrogen species, which is an inflammatory mediator involved in the induction of inflammation in cells, whereby a cosmetic composition comprising 3-hydroxypropionic acid or a derivative thereof as an effective ingredient can be used for anti-inflammatory purposes.

In the above applications, the effective ingredient, i.e., 3-hydroxypropionic acid or a derivative thereof, can be used in an amount of 0.01 wt.% to 1.5 wt.% based on the total weight of the cosmetic composition.

In order to ensure that the anti-inflammatory effect can be sufficiently achieved by the action of the active ingredient, the effective ingredient is preferably used in an amount of 0.01 wt.% or more or 0.012 wt.% or more based on the total weight of the cosmetic composition. However, if the effective ingredient is used in an excessive amount, the anti-inflammatory effect may be rather reduced or side effects such as cytotoxicity may appear. Therefore, the effective ingredient is preferably used in an amount of 1.5 wt.% or less, or 1.25 wt.% or less, or 1.1 wt.% or less, or 1.0 wt.% or less, based on the total weight of the cosmetic composition. Preferably, the effective ingredient may be used in an amount of 0.01 wt.% to 1.5 wt.%, or 0.01 wt.% to 1.25 wt.%, or 0.01 wt.% to 1.1 wt.%, or 0.01 wt.% to 1.0 wt.%, or 0.012 wt.% to 1.0 wt.%, based on the total weight of the cosmetic composition.

According to yet another embodiment of the present disclosure, there is provided a method of inhibiting the production of reactive nitrogen species, which is an inflammatory mediator, by using a cosmetic composition comprising 3-hydroxypropionic acid or a derivative thereof as an effective ingredient.

Specifically, a method for scavenging 2,2-diphenyl-1-picrylhydrazyl radicals using a cosmetic composition comprising 3-hydroxypropionic acid or a derivative thereof as an effective ingredient is provided.

Preferably, the method of inhibiting the production of reactive nitrogen species can be performed by using the above-mentioned cosmetic composition having anti-inflammatory activity. The method can be performed by applying the cosmetic composition to a human body site in need of anti-inflammatory activities. The method can be performed with an application frequency of one to several times a day (for example, one to five times or one to three times) for one to six months, one to seven days a week.

According to yet another embodiment of the present disclosure, there is provided a pharmaceutical composition having anti-inflammatory activity, comprising 3-hydroxypropionic acid or a derivative thereof as an active ingredient.

As a result of continuous research, the present inventors have found that 3-hydroxypropionic acid can exhibit excellent anti-inflammatory effects such as preventing, treating or ameliorating symptoms caused by the induction of inflammation in cells.

In particular, 3-hydroxypropionic acid inhibits the production of reactive nitrogen species, which is an inflammatory mediator involved in the induction of inflammation in cells, whereby a pharmaceutical composition comprising 3-hydroxypropionic acid or a derivative thereof as an active ingredient can exhibit excellent anti-inflammatory effects.

The pharmaceutical composition having anti-inflammatory activity comprises 3-hydroxypropionic acid or a derivative thereof as an active ingredient. Preferably, the pharmaceutical composition having anti-inflammatory activity comprises 3-hydroxypropionic acid as an active ingredient.

3-Hydroxypropionic acid synthesized by a chemical method or a biological method can be applied as an active ingredient.

According to an embodiment, the effective ingredient may be preferably 3-hydroxypropionic acid obtained through a biological process such as fermenting a strain having 3-hydroxypropionic acid production ability. The 3-hydroxypropionic acid obtained by the above method may include a radioactive carbon isotope (¹⁴C).

Preferably, the active ingredient may have a radioactive carbon isotope content of 20 pMC (percent modern carbon) or more and a biocarbon content of 20 wt.% or more as measured according to ASTM D6866-21 standard. Specifically, the active ingredient may have a radioactive carbon isotope content of 20 pMC or more, or 50 pMC or more, or 90 pMC or more, or 100 pMC or more, or 90 to 110 pMC, and a biocarbon content of 20 wt.% or more, or 50 wt.% or more, or 80 wt.% or more, or 90 wt.% or more, or 95 wt.% or more, or 90 to 100 wt.%, as measured in accordance with ASTM D6866-21 standard.

The pharmaceutical composition can inhibit the production of reactive nitrogen species, which is an inflammatory mediator due to the action of the effective ingredient. Thereby, the pharmaceutical composition can exhibit excellent anti-inflammatory effects, such as preventing, treating or ameliorating symptoms caused by the induction of inflammation in cells.

Here, the active nitrogen species is at least one compound selected from the group consisting of nitric oxide(NO), nitrate(NO₃⁻), nitrite(NO₂⁻), peroxynitrite(ONOO⁻), and 3-nitrotyrtyrosine.

According to an embodiment, the pharmaceutical composition can contain 0.01 wt.% to 1.5 wt.% of the effective ingredient based on the total weight of the pharmaceutical composition.

Specifically, the pharmaceutical composition can contain 0.01 wt.% or more or 0.012 wt.% or more; and 1.5 wt.% or less, or 1.25 wt.% or less, or 1.1 wt.% or less, or 1.0 wt.% or less of the effective ingredient based on the total weight of the pharmaceutical composition.

In order to ensure that the anti-inflammatory effect can be sufficiently achieved by the action of the active ingredient, the pharmaceutical composition preferably contains 0.01 wt.% or more or 0.012 wt.% or more of the effective ingredient.

However, if the effective ingredient is included in an excessive amount, the anti-inflammatory effect may be rather reduced or side effects such as cytotoxicity may appear. Therefore, the pharmaceutical composition preferably contains 1.5 wt.% or less, or 1.25 wt.% or less, or 1.1 wt.% or less, or 1.0 wt.% or less of the effective ingredient.

Preferably, the pharmaceutical composition may comprise 0.01 wt.% to 1.5 wt.%, or 0.01 wt.% to 1.25 wt.%, or 0.01 wt.% to 1.1 wt.%, or 0.01 wt.% to 1.0 wt.%, or 0.012 wt.% to 1.0 wt.% of the effective ingredient based on the total weight of the pharmaceutical composition.

The pharmaceutical composition may further comprise a pharmaceutically acceptable carrier.

As the carrier, those commonly used in the preparation of the pharmaceutical composition may be applied without particular limitation. As an example, the carrier may be sucrose, sorbitol, mannitol, lactose, dextrose, starch, gum acacia, calcium phosphate, alginate, gelatin, calcium silicate, microcrystalline cellulose, polyvinylpyrrolidone, cellulose, water, syrup, methyl cellulose, propyl 4-hydroxybenzoate, talc, magnesium stearate, mineral oil, and the like. Pharmaceutically acceptable carriers and formulations are described in detail in Remington's The Science and Practice of Pharmacy (23rd Edition, 2020).

The pharmaceutical composition may be orally or parenterally administered. In case of the parenteral administration, this may be administered through an application to skin, an intravenous injection, a subcutaneous injection, an intramuscular injection, an intraperitoneal injection, a dermal administration, etc.

An appropriate dosage of the pharmaceutical composition may be determined in comprehensive consideration of factors such as a patient's age, weight, gender, a formulation method, an administration method, administration route, and administration time.

The pharmaceutical composition can be formulated by applying a pharmaceutically acceptable carrier and/or excipient. The formulation of the pharmaceutical composition may be a solution, suspension, emulsion, ointment, powder, granule, tablet, or capsule in an aqueous medium or oil.

According to yet another embodiment of the present disclosure, there is provided use of 3-hydroxypropionic acid or a derivative thereof for the preparation of medicines having anti-inflammatory activity.

Preferably, the use of 3-hydroxypropionic acid or a derivative thereof for the preparation of the pharmaceutical composition having anti-inflammatory activity is provided.

3-Hydroxypropionic acid has the effect of inhibiting the production of reactive nitrogen species, which is an inflammatory mediator involved in the induction of inflammation in cells, whereby a pharmaceutical composition comprising 3-hydroxypropionic acid or a derivative thereof as an effective ingredient can be used for anti-inflammatory purposes.

In the above applications, the effective ingredient, i.e., 3-hydroxypropionic acid or a derivative thereof, can be used in an amount of 0.01 wt.% to 1.5 wt.% based on the total weight of the pharmaceutical composition.

In order to ensure that the anti-inflammatory effect can be sufficiently achieved by the action of the active ingredient, the effective ingredient is preferably used in an amount of 0.01 wt.% or more or 0.012 wt.% or more based on the total weight of the pharmaceutical composition. However, if the effective ingredient is used in an excessive amount, the anti-inflammatory effect may be rather reduced or side effects such as cytotoxicity may appear. Therefore, the effective ingredient is preferably used in an amount of 1.5 wt.% or less, or 1.25 wt.% or less, or 1.1 wt.% or less, or 1.0 wt.% or less, based on the total weight of the pharmaceutical composition. Preferably, the effective ingredient may be used in an amount of 0.01 wt.% to 1.5 wt.%, or 0.01 wt.% to 1.25 wt.%, or 0.01 wt.% to 1.1 wt.%, or 0.01 wt.% to 1.0 wt.%, or 0.012 wt.% to 1.0 wt.%, based on the total weight of the pharmaceutical composition.

According to yet another embodiment of the present disclosure, there is provided a method of inhibiting the production of reactive nitrogen species, which is an inflammatory mediator, by using the pharmaceutical composition, comprising 3-hydroxypropionic acid or a derivative thereof as an active ingredient.

Preferably, the method of inhibiting the production of reactive nitrogen species can be performed by using the above-mentioned pharmaceutical composition having anti-inflammatory activity. The method can be performed orally or parenterally. The method can be performed with an application frequency of one to several times a day (for example, one to five times or one to three times) for one to six months, one to seven days a week. The method may be performed according to a conventional procedure in comprehensive consideration of factors such as a patient's age, weight, gender, formulation of 3-hydroxypropionic acid or a derivative thereof, an administration method, and administration time.

### [ADVANTAGEOUS EFFECTS]

According to the present disclosure, a cosmetic composition and a pharmaceutical composition that can inhibit the production of reactive nitrogen species, which is an inflammatory mediator involved in the induction of inflammation in cells are provided.

### [DETAILED DESCRIPTION OF THE EMBODIMENTS]

Hereinafter, actions and effects of the invention will be described in more detail with reference to specific examples. However, the following examples are provided to assist the understanding of the invention, and are not intended to limit the scope of the invention in any way. It would be obvious to those skilled in the art that various changes and modifications can be made without departing from the sprit and scope of the invention.

### Preparation Example 1

Prepared was a recombinant vector incorporating a gene encoding glycerol dehydratase and aldehyde dehydrogenase, which are known to produce 3-hydroxypropionic acid using glycerol as a substrate. The prepared recombinant vector was introduced into the *E. coli* W3110 strain to prepare a 3-hydroxypropionic acid-producing strain.

Specifically, pCDF_J23101_dhaB_gdrAB_J23100_aldH_btuR vector, was obtained by cloning BtuR gene encoding adenosyltransferase into plasmid pCDF including a gene(dhaB) encoding glycerol dehydratase, a gene(aldH) encoding aldehyde dehydrogenase and a gene(gdrAB) encoding glycerol dehydratase reactivase. The vector was introduced into W3110 strain (KCCM 40219) by electroporation using an electroporation device (Bio-Rad, Gene Pulser Xcell) to prepare a 3-hydroxypropionic acid-producing strain.

The 3-hydroxypropionic acid-producing strain was fermented and cultured at 35°C in a 5 L fermenter by using unpurified glycerol as a carbon source, so as to produce 3-hydroxypropionic acid. In order to prevent a decrease in pH due to the production of 3-hydroxypropionic acid, calcium hydroxide (Ca(OH)₂), an alkali metal salt, was added to maintain a neutral pH during fermentation.

After fermentation culture, cells were removed by centrifugation (4000 rpm, 10 minutes, 4°C), and primary purification was performed by using activated carbon. The concentration of 3-hydroxypropionic acid in the fermented solution obtained after the primary purification was at a level of 50 to 100 g/L, and the fermented solution was concentrated at a concentration of 600 g/L using a rotary evaporator (50°C, 50 mbar) to prepare a concentrate, which was then stirred at room temperature (300 rpm) to produce Ca(3HP)₂ crystals.

The resulting crystals were washed three times with ethanol and dried in an oven at 50°C to finally recover crystals. The weight of the crystals was measured, the crystals were dissolved in water to obtain 3-hydroxypropionic acid(3HP) using high performance liquid chromatography(HPLC).

### Preparation Example 2

3-Hydroxypropionic acid(3HP) was obtained in substantially the same manner as in Preparation Example 1, except for using Mg(OH)₂ instead of Ca(OH)₂ as an alkali metal salt.

### Example 1

0.0125 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a lotion.

### Example 2

0.025 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a lotion.

### Example 3

0.05 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a toner emulsion.

### Example 4

0.1 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a toner emulsion.

### Example 5

0.0125 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt%) were mixed to prepare a pharmaceutical composition as an ointment formulation.

### Example 6

0.025 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a pharmaceutical composition as an ointment formulation.

### Example 7

0.05 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a pharmaceutical composition as an emulsion formulation

### Example 8

0.1 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a pharmaceutical composition as an emulsion formulation.

### Experimental Example 1

### (Measurement of radioactive carbon isotope and biocarbon content)

The radioactive carbon isotope content(pMC) and biocarbon content of the 3-hydroxypropionic acid obtained from Preparation Examples 1 and 2 were measured according to ASTM D6866-21 (Method B).

The biocarbon content means the content of biocarbon contained in the 3-hydroxypropionate crystals obtained from Preparation Examples 1 and 2, and the radioactive carbon isotope ratio(pMC) refers to a ratio of the radioactive carbon isotope (¹⁴C) contained in the 3-hydroxypropionate crystals to the radioactive carbon isotope (¹⁴C) of the modern reference material.

**[Table 1]**

| | Concentration (g/L) | Radioactive carbon isotope ratio (pMC) | Biocarbon content (wt.%) |
|---|---|---|---|
| Preparation Example 1 | 600 | 101.52 | 100 |
| Preparation Example 2 | 800 | 102.23 | 100 |

Referring to Table 1, it was confirmed that both of the 3HP salt crystals of Preparation Examples 1 and 2 have a radioactive carbon isotope ratio of 101 pMC or more and a biocarbon content of 100 wt.%.

### Experimental Example 2

### (Evaluation of reactive nitrogen species inhibiting ability)

Mouse macrophages were divided into 1X10⁶ cells in a 6-well plate and cultured for 24 hours, then treated with LPS (Lipopolysaccharide, concentration 1 *µ*g/ml) solution, and treated with the 3-hydroxypropionic acid aqueous solution according to Preparation Example 1 at four concentrations (3HP concentration: 0 wt.%, 0.0125 wt.%, 0.025 wt.%, 0.05 wt.%), and then cultured again for 24 hours. Only the medium was then separated and centrifuged for 10 minutes to collect only the supernatant. The supernatant was mixed with Griess reagent (Sigma-Aldrich) reagent in a 1:1 ratio and reacted at room temperature, and then the absorbance (OD, wavelength 540 nm) was measured using a microplate reader. The measured value was substituted into the regression equation obtained from the standard curve prepared by reacting Griess reagent (Sigma-Aldrich) and NaNO₂ in a 1:1 ratio to calculate the amount of nitric oxide(NO) produced. Here, the case in which neither the LPS solution nor the 3HP aqueous solution was treated was performed as a negative control group.

**[Table 2]**

| 3HP concentration (wt.%) | Nitrogen oxide concentration (µM) | | | | | Significance probability relative to negative control group (p-value) |
|---|---|---|---|---|---|---|
| | Once | Two times | Three times | Average | Standard deviation | |
| Negative control group | -14.534 | -14.718 | -14.903 | -14.718 | 0.185 | - |
| 0 | 70.288 | 70.129 | 71.185 | 70.534 | 0.569 | - |
| 0.0125 | 63.956 | 64.800 | 66.093 | 64.949 | 1.076 | 0.005 |
| 0.025 | 63.138 | 65.222 | 65.961 | 64.774 | 1.464 | 0.014 |
| 0.05 | 56.146 | 56.357 | 57.386 | 56.630 | 0.663 | 0.000 |

Referring to the experimental results, it was confirmed that the effect of inhibiting the production of reactive nitrogen species was exhibited under the treatment concentration of the 3-hydroxypropionic acid.

### Formulation Example 1

0.2 wt.% of 3-hydroxypropionic acid obtained from Preparation Example 1, 3.0 wt.% of glycerin, 2.0 wt.% of butylene glycol, 2.0 wt.% of propylene glycol, 0.1 wt.% of polyacrylic acid, 0.2 wt.% of polyethylene glycol-12 nonylphenyl ether, 0.4 wt.% of polysorbate-80, 10.0 wt.% of ethanol, 0.1 wt.% of triethanolamine, and the remaining amount of purified water were mixed to prepare a softening lotion (skin lotion).

### Formulation Example 2

1.0 wt.% of 3-hydroxypropionic acid obtained from Preparation Example 1, 3.0 wt.% of glycerin, 3.0 wt.% of butylene glycol, 0.5 wt.% of liquid paraffin, 4.0 wt.% of beeswax, 5.0 wt.% of caprylic/capric triglyceride, 5.0 wt.% of squalene, 1.5 wt.% of polysorbate-60, 1.0 wt.% of cetearyl alcohol, 1.5 wt.% of sorbitan sesquioleate, 0.1 wt.% of polyacrylic acid, 0.2 wt.% of triethanol amine, and the remaining amount of purified water were mixed to prepare a nourishing lotion (milk lotion).

### Formulation Example 3

2.0 wt.% of 3-hydroxypropionic acid obtained from Preparation Example 1, 3.0 wt.% of glycerin, 3.0 wt.% of butylene glycol, 7.0 wt.% of liquid paraffin, 7.0 wt.% of beta-glucan, 3.0 wt.% of caprylic/capric triglyceride, 5.0 wt.% of squalene, 1.5 wt.% of cetearyl glucoside, 1.2 wt.% of polysorbate-60, 0.4 wt.% of sorbitan stearate, 0.1 wt.% of polyacrylic acid, 0.1 wt.% of triethanol amine, and the remaining amount of purified water were mixed to prepare a nourishing cream.

### Formulation Example 4

8 mg of 3-hydroxypropionic acid obtained from Preparation Example 1, 9 mg of vitamin C, 9 mg of vitamin E, 200 mg of lactose, and 200 mg of galactooligosaccharide were mixed, and granulated using a fluidized bed dryer, and 5 mg of sugar ester was added to prepare a tablet composition. 500 mg of the tablet composition was tableted to prepare tablets.

### Formulation Example 5

8 mg of 3-hydroxypropionic acid obtained from Preparation Example 1, 9 mg of vitamin C, 9 mg of vitamin E, 10 mg of vegetable hardened oil, and 9 mg of lecithin were mixed to prepare a soft capsule filling liquid. Separately, 65 wt.% of gelatin, 25 wt.% of glycerin, and 10 wt.% of sorbitol solution were mixed to prepare a soft capsule sheet. The soft capsule filling liquid was filled at 400 mg per capsule to prepare a soft capsule.

### Formulation Example 6

8 mg of 3-hydroxypropionic acid obtained from Preparation Example 1, 9 mg of vitamin C, 9 mg of vitamin E, 500 mg of starch, and 200 mg of anhydrous crystalline glucose were mixed, formed into granules using a fluidized bed granulator, and then filled into a capsule to prepare a granule formulation.

### Formulation Example 7

8 mg of 3-hydroxypropionic acid obtained from Preparation Example 1, 9 mg of vitamin C, 9 mg of vitamin E, 0.7 g of citric acid, 10 g of glucose, and 25 g of liquid oligosaccharide were mixed and then 300 ml of purified water were added to prepare a drink composition. The drink composition was filled into bottles at 200 ml each and sterilized at 130°C for about 5 seconds to prepare a drink formulation.

### Formulation Example 8

30 mg of 3-hydroxypropionic acid obtained from Preparation Example 1, an appropriate amount of sterilized distilled water for injection, and an appropriate amount of a pH regulator were mixed to prepare an injection formulation.

While the present disclosure has been described with reference to limited embodiments, the present disclosure should be not limited to the disclosed embodiment and it will be apparent to those skilled in the art that various modifications and variations may be made without departing from the scope of the technical idea of the present disclosure and the scope of the appended claims and their equivalents.

## Claims

1. A cosmetic composition having anti-inflammatory activity, comprising 3-hydroxypropionic acid or a derivative thereof as an effective ingredient.

2. The cosmetic composition having anti-inflammatory activity according to claim 1, wherein the cosmetic composition inhibits the production of reactive nitrogen species.

3. The cosmetic composition having anti-inflammatory activity according to claim 1, wherein the cosmetic composition comprises 0.01 wt.% to 1 wt.% of the effective ingredient based on the total weight of the composition.

4. The cosmetic composition having anti-inflammatory activity according to claim 1, wherein the effective ingredient has a radioactive carbon isotope content of 20 pMC (percent modern carbon) or more and a biocarbon content of 20 wt.% or more as measured according to ASTM D6866-21 standard.

5. The cosmetic composition having anti-inflammatory activity according to claim 1, wherein the cosmetic composition further comprises a physiologically acceptable medium.

6. The cosmetic composition having anti-inflammatory activity according to claim 1, wherein the cosmetic composition has a formulation that includes a solution, a suspension, an emulsion, a paste, a gel, a cream, a lotion, a powder, an oil, a foam, a pack, a spray, an aerosol, a balm, a solid soap, a liquid soap, a powder foundation, an emulsion foundation, or a wax foundation.

7. A pharmaceutical composition having anti-inflammatory activity, comprising 3-hydroxypropionic acid or a derivative thereof as an effective ingredient.

8. The pharmaceutical composition having anti-inflammatory activity according to claim 7, wherein the pharmaceutical composition inhibits the production of reactive nitrogen species.

9. The pharmaceutical composition having anti-inflammatory activity according to claim 7, wherein the pharmaceutical composition comprises 0.01 wt.% to 1 wt.% of the effective ingredient based on the total weight of the composition.

10. The pharmaceutical composition having anti-inflammatory activity according to claim 7, wherein the effective ingredient has a radioactive carbon isotope content of 20 pMC (percent modern carbon) or more and a biocarbon content of 20 wt.% or more as measured according to ASTM D6866-21 standard.

11. The pharmaceutical composition having anti-inflammatory activity according to claim 7, wherein the pharmaceutical composition further comprises a pharmaceutically acceptable carrier.

12. The pharmaceutical composition having anti-inflammatory activity according to claim 7, wherein the pharmaceutical composition is for oral or parenteral use.

13. Use of 3-hydroxypropionic acid or a derivative thereof for the preparation of cosmetics having anti-inflammatory activity or medicines having anti-inflammatory activity.

14. A method of inhibiting the production of reactive nitrogen species, which is an inflammatory mediator, by using a cosmetic composition or a pharmaceutical composition, comprising 3-hydroxypropionic acid or a derivative thereof as an active ingredient.
